# EUROPÄISCHE PATENTSCHRIFT

(11) **EP 1 748 797 B1**
(45) Veröffentlichungstag und Bekanntmachung des Hinweises auf die Patenterteilung: **27.08.2008**
(21) Anmeldenummer: 05737904.2
(22) Anmeldetag: 04.05.2005
(51) Int. Cl.: A61L 2/18, A61K 31/155, A61K 31/405, A01N 47/44, A61M 1/16

(54) **VERWENDUNG VON BIGUANIDEN UND VERFAHREN ZUR DESINFEKTION**
USE OF BIGUANIDES AND METHOD OF STERILISATION
UTILISATION DE BIGUANIDES ET PROCEDE DE STERILISATION

(30) Priorität: 14.05.2004 DE 102004024140
(43) Veröffentlichungstag der Anmeldung: 07.02.2007
(73) Patentinhaber: Fresenius Medical Care Deutschland GmbH, 61352 Bad Homburg (DE)
(72) Erfinder: LEINENBACH, Hans-Peter, 66636 Tholey (DE); KUHN, Stefan, 66538 Neunkirchen (DE); GABSDIL, Ute, 69168 Wiesloch (DE)
(74) Vertreter: Weiß, Stefan
(86) Internationale Anmeldenummer: PCT/EP2005/004836
(87) Internationale Veröffentlichungsnummer: WO 2005/110498

(56) Entgegenhaltungen:
- WO-A-00/18442
- WO-A-94/27440
- WO-A-02/051464
- DE-A- 19 503 613
- GB-A- 1 533 255
- US-A- 5 078 967
- US-A- 5 498 416
- US-A1- 2004 185 028
- ZHANG Y ET AL: "Synthesis and antimicrobial activity of polymeric guanidine and biguanidine salts" POLYMER, ELSEVIER SCIENCE PUBLISHERS B.V, GB, Bd. 40, Nr. 22, Oktober 1999 (1999-10), Seiten 6189-6198, XP004172488 ISSN: 0032-3861

## Beschreibung

Die Erfindung betrifft das Gebiet der Desinfektion und Konservierung von Oberflächen einer proteinhaltigen medizinischen Vorrichtung ansgewählt aus der Gruppe Dialysator, Blutfilter und Adsorber.

Verschiedene medizinische Vorrichtungen kommen während der Benutzung mit biologischen Flüssigkeiten in Kontakt. Dies ist insbesondere der Fall bei medizinischen Vorrichtungen, die im extrakorporalen Blutkreislauf eingesetzt werden. Zu diesen Vorrichtungen zählen Dialysatoren, Blutfilter und Adsorber. In allen Fällen, in denen eine Flüssigkeit nach dem Kontakt mit der medizinischen Vorrichtung Menschen oder Tieren reinfundiert wird, ist es essentiell, dass die mit der Flüssigkeit in Kontakt tretenden Oberflächen pathogenfrei sind. In den meisten Fällen wird dies durch eine Hitzesterilisation der medizinischen Vorrichtung erreicht. In anderen Fällen kann eine Hitzesterilisation nicht effektiv durchgeführt werden, weil durch eine solche Sterilisation die Funktionalität der medizinischen Vorrichtung beeinträchtigt würde. Dies ist vor allem bei Adsorbern der Fall, die Proteine als Liganden gebunden haben.

Solche Adsorber dieser Art sind aus dem Stand der Technik bekannt. So wird in EP 0 222 146 ein Adsorbens zur Entfernung von Antigen-Antikörper-Komplexen beschrieben. Als Ligand fungiert hier das Protein C1q.
Hämodialysatoren werden zum Teil nach der ersten Behandlung wiederverwendet. Auch in diesen Fällen ist es notwendig den Dialysator durch eine effektive Dekontamination vor dem nächsten Einsatz zu regenerieren. Dies kann wie in US 5,192,459 durch das Waschen mit einem Desinfektionsmittel geschehen. Als Desinfektionsmittel werden verschiedene Säuren vorgeschlagen. In WO 00/27439 dagegen werden Oxidationsmittel in Gegenwart von Harnstoff als Desinfektionsmittel eingesetzt.

Keine der genannten Methoden kann allerdings für eine effektive Dekontamination eines proteinhaltigen Adsorbens unter Erhaltung der Funktionalität eingesetzt werden. Bisher wurden daher zu diesem Zweck Mittel eingesetzt, die Schwermetalle oder Natriumazid enthielten und somit geringe Biokompatibilität aufwiesen. Die aufwändige vollständige Entfernung dieser Desinfektionsmittel nach dem Stand der Technik ist daher vor Gebrauch der medizinischen Vorrichtung absolut notwendig.

Aufgabe der Erfindung ist es ein Desinfektions-und Konservierungsmittel zur Verfügung zu stellen, das eine effektive Dekontamination von medizinischen Vorrichtungen, insbesondere medizinischen Vorrichtungen zur Verwendung im extrakorporalen Kreislauf ermöglicht, und gleichzeitig toxikologisch unbedenklich ist.
Die genannten Aufgaben werden erfindungsgemäß durch eine Verwendung einer Biguanidhaltigen Lösung nach Anspruch 1 gelöst. Weitere vorteilhafte Ausführungsformen sind Gegenstand der Unteransprüche.

Die Erfindung beruht auf der überraschenden Erkenntnis, dass sich Lösungen enthaltend eine Biguanid-Verbindung ausgezeichnet zur Desinfektion von medizinischen Vorrichtungen zur Verwendung im extrakorporalen Blutkreislauf eignen. Diese Lösungen die zuvor als Wundantiseptika und Konservierungsmittel für Ophthalmika eingesetzt wurde, zeichnet sich durch eine hervorragende Biokompatibilität und eine geringe Toxizität für den Menschen aus. Die Lösung kann dennoch vergleichbare oder sogar bessere Ergebnisse bei der Desinfektion und Konservierung von genannten medizinischen Geräten erzielen wie die zuvor im Stand der Technik verwendeten Substanzen höherer Toxizität.

Biguanidin-haltige Lösungen sind im Stand der Technik als Wundantiseptika bekannt (EP 0 450 117). Die Verwendung von Biguaniden zur Beschichtung von medizinischen Vorrichtungen zur extrakorporalen Behandlung wurde in EP 0456 093 beschrieben. Als besonders effektives Desinfektionsmittel wird Poly(hexamethylen)biguanid mit einem Molekulargewicht von 2900 bis 15000 in EP 0 700 249 ausgewiesen. Dieses Antiinfektivum wird ebenfalls zur Wundbehandlung von der Firma Fresenius Kabi unter der Bezeichnung LAVASEPT^{®} vertrieben. Als eine weitere Anwendung einer solchen Lösung ist die Desinfektion und Konservierung von Kontaktlinsen bekannt.

Im Rahmen der Erfindung wird bevorzugt Poly(hexymethylen)biguanid mit einem Molekulargewicht von 2900 bis 15000 (PHMB) als Biguanid-Verbindung verwendet. Eine besonders bevorzugte Desinfektionslösung enthält PHMB in einer Konzentration von 0,01 bis 0,5 Gew-% wobei Konzentrationen von 0,02 bis 0,06 Gew-% besonders vorteilhaft sind. Als Medium dient ein neutraler phosphathaltiger Puffer der bevorzugt folgende Substanzen enthalten kann: Natriumzitrat Dihydrat, Natriumacetat Trihydrat, Natriumchlorid, Natriummonohydrogenphosphat, Kaliumdihydrogenphosphat. Eine solche Lösung desinfiziert und konserviert vorgenannte medizinische Vorrichtungen wirkungsvoll und genügt den Anforderungen des in USP 27 (United States Pharmacopeia) beschriebenen "antimicrobial effectiveness tests".

Ein weiterer wichtiger Vorteil der erfindungsgemäßen Lösung ist der Erhalt der Funktionalität von Proteinen. Es konnte weiterhin gezeigt werden, dass nach einer Füllung eines proteinhaltigen Adsorbers mit der oben beschriebenen Lösung als Konservierungsmittel vor einem Lagerungszeitraum von 4 Wochen bei Dunkelheit und bei einer Temperatur von 37°C kein Funktionalitätsverlust der Proteine zu erkennen war. Bei einer Lagerung bei 4°C ist eine wesentlich längere Lagerung ohne Funktionalitätsverlust möglich. Die Erfindung umfasst daher auch ein Verfahren zur Desinfektion und Konservierung von Oberflächen einer medizinischen Vorrichtung unter Verwendung einer Lösung nach Anspruch 1 mit den Schritten die medizinische Vorrichtung mit der Lösung enthaltend eine Biguanid-Verbindung zu füllen und die medizinische Vorrichtung dann in gefülltem Zustand zu lagern.

Weiterhin ist vorteilhaft an der erfindungsgemäßen Verwendung der Lösung, dass sich das Konservierungsmittel vor Gebrauch der medizinischen Vorrichtung in einem extrakorporalen Kreislauf vollständig ausspülen lässt. Zur Überprüfung der Vollständigkeit des Ausspülvorgangs der erfindungsgemäßen Lösung nach Anspruch 1 ist es ein weiterer Aspekt der Erfindung, der Lösung ein nicht-toxisches Additiv hinzuzufügen, das sich spektroskopisch nachweisen lässt. Die Erfindung umfasst daher auch eine Lösung zur Desinfektion oder Konservierung von medizinischen Vorrichtungen **dadurch gekennzeichnet, dass** sie eine Biguanid-Verbindung und ein spektroskopisch aktives, nicht-toxisches Additiv enthält. In einer besonders bevorzugten Ausführungsform wird Tryptophan in einer Konzentration von mindestens 0,005 Gew-% als Additiv verwendet. Besonders bevorzugt wird Tryptophan in einer Konzentration von 0,01 bis 0,1 Gew-% zugesetzt. Tryptophan adsorbiert UV-Licht bei einer Wellenlänge von 280 nm und eine Konzentrationsbestimmung in einer Lösung kann spektroskopisch anhand des Gesetzes von Lambert-Beer durchgeführt werden. Tryptophan besitzt selbst keine desinfizierende Wirkung. Es wird daher ausschließlich zur Messung des Fortschritts des Ausspülvorgangs und eines Konservierungsschrittes eingesetzt, welcher sich der Behandlung anschließt. In Versuchen konnte gezeigt werden, dass Tryptophan im gleichen Maße wie PHMB bei dem Ausspülvorgang von der Säule entfernt wurde.

Im Folgenden werden Beispiele vorgestellt, die zur Erläuterung der Erfindung beitragen sollen, ohne die Erfindung in irgend einer Weise einzuschränken.

### Beispiele

### Beispiel 1: Zusammensetzung der Lösung

Eine Lösung mit folgenden Bestandteilen wird als Desinfektions-und Konservierungsmittelmittel für einen Adsorber hergestellt:
400 mg Poly(hexamethylen)biguanid
300 mg Tryptophan
3,3 g Natriumzitrat Dihydrat
5,44 g Natriumacetat Trihydrat
4,92 g Natriumchlorid
2,9 g Dinatriumhydrogenphosphat * 12 H₂0
260 mg Kaliumdihydrogenphosphat
die Bestandteile werden gelöst, die Lösung wird auf einen pH von 7,0 eingestellt und auf 1 Liter aufgefüllt.

### Beispiel 2: Desinfizierende Wirkung

Eine Adsorbersäule des Typs IMMUNOSORBA (Fresenius Adsorber Technology GmbH) wird zur Entfernung von Antikörpern aus Blutplasma verwendet und bindet die Antikörper über Protein A von Staphylococcus aureus. Diese Adsorbersäule wird mit einer Lösung nach Beispiel 1 befüllt. Die desinfizierende / konservierende Wirkung der Lösung entspricht den Vorgaben der USP 27.

### Beispiel 3: Funktionalitätstest nach Lagerung

Nach Beispiel 2 befüllte Adsorbersäulen werden über einen längeren Zeitraum lichtfrei eingelagert. Bei einer Lagerung von bei 40°C konnte vor einem Zeitraum von 4 Wochen kein Funktionalitätsverlust festgestellt werden. Nach einer 35-wöchigen Lagerung bei 37°C war die Ig-Bindungskapazität der Säule für Antikörper um 20% reduziert.. Bei einer 46-wöchigen Lagerung bei 4°C zeigte auch die nach Beispiel 2 befüllte Säule keinen Funktionalitätsverlust.

### Beispiel 4: Ausspülbarkeit der Konservierungslösung

Eine nach Beispiel 2 befüllte Adsorbersäule wird an einen Apparat des Typs CITEM-10 (Fresenius Adsorber Technology) angeschlossen. Die Säule wird durch ein vorgesehenes Ausspülprogramm des Apparats ausgespült.
Dabei wird die Säule zunächst mit 300 ml eines Puffers 1 der folgenden Zusammensetzung gespült:
3,3 g Natriumzitrat Dihydrat
5,44 g Natriumacetat Trihydrat
4,92 g Natriumchlorid
2,9 g Dinatriumhydrogenphosphat * 12 H₂0
260 mg Kaliumdihydrogenphosphat
pH 7,0
anschließend mit 100 ml eines Eluants der folgenden Zusammensetzung:
3,3 g Natriumzitrat Dihydrat
5,44 g Natriumacetat Trihydrat
4,92 g Natriumchlorid
2,9 g Dinatriumhydrogenphosphat * 12 H₂0
260 mg Kaliumdihydrogenphosphat
pH 2,0
und abschließend wiederum mit weiteren 350 ml des Puffers 1.

Nach dem Ausspülen wurde eine Behandlungssimulation mit 3 Liter Blutplasma durchgeführt.
Der Ausspülvorgang der Konservierungslösung innerhalb des Säulenvorbereitungsschrittes wird mittels UV-Detektion der Aminosäure Tryptohan am CITEM 10 Monitor überwacht.

Folgende Ergebnisse wurden beobachtet:
a) Am Ende des oben beschriebenen Ausspülvorgangs wurden keine Spuren von PHMB im nachfolgend behandelten Plasma oberhalb der Nachweisgrenze nachgewiesen (LOD PHMB = 0,90mg/Liter)
b) Im behandelten Plasma konnten Tryptophankonzentrationen nachgewiesen werden, welche zwischen der unteren Nachweisgrenze (2,2 ppm) und der Trp-Quantifizierungsgrenze (7,0 ppm) lagen
c) Es wurden ca. 20% mehr PHMB und ca. 8 % mehr Trp in den Spüllösungen gefunden, als die mittels angenommenem Volumen berechnete Menge. In nachfolgenden Untersuchungen konnte gezeigt werden, dass während der Konservierung 20% PHMB bzw. 8% Trp mehr auf den Säulen verbleiben. Weiterhin konnte in diesen Untersuchungen gezeigt werden, dass sich diese Anteile während des Säulenvorbereitungsschrittes vollständig ausspülen lassen.
d) Innerhalb der 20 Behandlungssimulationen blieb der CPI (Column Performance Index) auf hohen Niveau stabil (>35)
e) Die Bindungskapazitäten gegenüber den Antikörperklassen IgG, IgM, IgA und der Subklasse IgG3 blieben stabil
Trp zeigt ein dem PHMB sehr ähnliches Elutionsverhalten und eignet sich daher als Indikator des Fortschritts sowohl der Ausspülung vom PHMB, als auch des Konservierens der Säulen nach den Behandlungen.

Es konnte weiterhin kein Unterschied im Ausspülverhalten neuer und Plasma- belasteter Säulen festgestellt werden.

## Patentansprüche

1. Verwendung einer Lösung enthaltend eine Biguanidverbindung zur Desinfektion und Konservierung von Oberflächen einer proteinhaltigen medizinischen Vorrichtung, ausgewählt aus der Gruppe Dialysator, Blutfilter und Adsorber.

2. Verwendung nach Anspruch 1 **dadurch gekennzeichnet, dass** es sich bei der Biguanidverbindung um Poly(hexamethylen)biguanid handelt.

3. Verwendung nach Anspruch 2 **dadurch gekennzeichnet, dass** die Biguanidverbindung ein Molekulargewicht Mw von 2900 bis 15000 aufweist.

4. Verwendung nach Anspruch 1 **dadurch gekennzeichnet, dass** die Lösung eine Konzentration der Biguanidverbindung von 0,01 bis 0,5 Gew.-% enthält.

5. Verwendung nach Anspruch 1 **dadurch gekennzeichnet, dass** die Lösung Tryptophan enthält.

6. Verwendung nach Anspruch 5 **dadurch gekennzeichnet, dass** die Lösung eine Tryptophankonzentration mindestens 0,005 Gew.-% enthält.

7. Verwendung nach Anspruch 1 **dadurch gekennzeichnet, dass** die medizinische Vorrichtung eine Hämodialysator ist.

8. Verfahren zur Desinfektion und Konservierung von Oberflächen einer proteinhaltigen medizinischen Vorrichtung, ausgewählt aus der Gruppe Dialysator, Blutfilter und Adsorber,
**dadurch gekennzeichnet, dass**
die medizinische Vorrichtung mit der Lösung enthaltend eine Biguanid-Verbindung in Kontakt mit den Oberflächen gefüllt wird und
die medizinische Vorrichtung in gefülltem Zustand gelagert wird.

9. Verfahren nach Anspruch 8 **dadurch gekennzeichnet, dass** vor der Benutzung die Lösung aus der medizinischen Vorrichtung ausgespült wird.

10. Verfahren nach Anspruch 9 **dadurch gekennzeichnet, dass** die Lösung zusätzlich ein spektroskopisch aktives, nicht-toxisches Additiv enthält und der Fortschritt des Ausspülvorgangs durch spektroskopische Messungen des spektroskopisch aktiven, nicht-toxischen Additivs in der ausgespülten Lösung überprüft wird.

11. Verfahren nach Anspruch 10 **dadurch gekennzeichnet, dass** es sich bei dem Additiv um Tryptophan handelt.

## Claims

1. The use of a solution containing a biguanide compound for disinfection and preservation of surfaces of a protein-containing medical device selected from the group comprising a dialyzer, a blood filter and an adsorber.

2. The use as claimed in claim 1, **characterized in that** the biguanide compound is poly-(hexamethylene) biguanide.

3. The use as claimed in claim 2, **characterized in that** the biguanide compound has a molecular weight Mw of 2900 to 15000.

4. The use as claimed in claim 1, **characterized in that** the solution contains a concentration of the biguanide compound of 0.01 to 0.5 wt. %.

5. The use as claimed in claim 1, **characterized in that** the solution contains tryptophan.

6. The use as claimed in claim 5, **characterized in that** the solution contains a tryptophan concentration of at least 0.005 wt. %.

7. The use as claimed in claim 1, **characterized in that** the medical device is a hemodialyzer.

8. A method for disinfection and preservation of surfaces of a protein-containing medical device, selected from the group comprising a dialyzer, a blood filter and an adsorber,
**characterized in that**
the medical device is filled with the solution containing a biguanide compound in contact with the surfaces, and
the medical device is stored in the filled state.

9. The method as claimed in claim 8, **characterized in that** the solution is rinsed from the medical device before use.

10. The method as claimed in claim 9, **characterized in that** the solution additionally contains a spectroscopically active, non-toxic additive and the progress of the rinsing process is monitored by spectroscopic measurements of the spectroscopically active, non-toxic additives in the rinsed solution.

11. The method as claimed in claim 10, **characterized in that** the additive is tryptophan.

## Revendications

1. Utilisation d'une solution comprenant une liaison biguanide pour la désinfection et conservation de surface d'un dispositif médical contenant des protéines, sélectionné parmi le groupe de dialyseur, filtre à sang, et adsorbeur.

2. Utilisation selon la revendication 1 **caractérisé en ce qu'**il s'agit de poly(héxaméthylène)biguanide pour la liaison biguanide.

3. Utilisation selon la revendication 2 **caractérisé en ce que** la liaison biguanide a un poids moléculaire Mw de 2900 a 15000.

4. Utilisation selon la revendication 1 **caractérisé en ce que** la solution comprend une concentration de liaison biguanide de 0,01 à 0,5 % poids.

5. Utilisation selon la revendication 1 **caractérisé en ce que** la solution contient du tryptophane.

6. Utilisation selon la revendication 5 **caractérisé en ce que** la solution contient une concentration de tryptophane d'au moins 0,005 % poids.

7. Utilisation selon la revendication 1 **caractérisé en ce que** le dispositif médical est un hémodyaliseur.

8. Procédé pour la désinfection et la conservation de surface d'un dispositif médical contenant des protéines, sélectionné parmi le groupe de dialyseur, filtre à sang, et adsorbeur.
**caractérisé en ce que**
le dispositif médical est rempli avec la solution comprenant une liaison biguanide en contact avec les surfaces et
le dispositif médical est stocké dans l'état rempli.

9. Procédé selon la revendication 8 **caractérisé en ce qu'**avant l'utilisation la solution est rincée du dispositif médical.

10. Procédé selon la revendication 9 **caractérisé en ce que** la solution comprend en plus un additif actif de manière spectroscopique, non toxique et l'avancée du rinçage est contrôlé via mesures spectroscopiques de l'additif actif de manière spectroscopique, non toxique dans la solution rincée.

11. Procédé selon la revendication 10 **caractérisé en ce qu'**il s'agit de tryptophane pour l'additif.
